# EUROPEAN PATENT SPECIFICATION

(11) **EP 2 183 375 B1**
(45) Date of publication and mention of the grant of the patent: **24.06.2015**
(21) Application number: 08805419.2
(22) Date of filing: 05.09.2008
(51) Int. Cl.: C12P 7/64

(54) **PROCESS FOR THE FERMENTATIVE PRODUCTION OF A LIPID FROM A STARTING MATERIAL, WHICH COMPRISES ALCOHOL, SOAPS AND/OR FATTY ACIDS, INVOLVING A STEP OF PRECIPITATING ALKALINE EARTH METAL SOAPS**
VERFAHREN ZUR FERMENTATIVEN HERSTELLUNG EINES LIPIDS AUS EINEM AUSGANGSMATERIAL, DAS ALKOHOL, SEIFEN UND/ODER FETTSÄUREN ENTHÄLT, UMFASSEND EINEN SCHRITT DER AUSFÄLLUNG VON ERDALKALIMETALLSEIFEN
PRODUCTION PAR FERMENTATION D'UN LIPIDE À PARTIR D'UN MELANGE D'ALCOOL, DE SAVONS ET/OU D'ACIDES GRAS COMPRENANT UNE ÉTAPE DE PRECIPITATION DE SAVON DE MÉTAL ALCALINO-TERREUX

(30) Priority: 07.09.2007 FI 20075618; 07.09.2007 US 967795 P
(43) Date of publication of application: 12.05.2010
(73) Proprietor: Aalto University Foundation, 00076 Aalto (FI)
(72) Inventor: PASTINEN, Ossi, FI-02460 Kantvik (FI); LAAKSO, Simo, FI-20380 Turku (FI); HOKKANEN, Sanna, FI-02780 Espoo (FI); VAHVASELKÄ, Marjatta, FI-00400 Helsinki (FI)
(74) Representative: Seppo Laine Oy
(86) International application number: PCT/FI2008/050496
(87) International publication number: WO 2009/030821

(56) References cited:
- PYLE, D. & WEN, Z.: "Production of Omega-3 Polyunsaturated Fatty Acid from Biodiesel-Waste Glycerol by Microalgal Fermentation" 2007 ASABE ANNUAL INTERNATIONAL MEETING, TECHNICAL PAPERS, AMERICAN SOCIETY OF AGRICULTURAL AND BIOLOGICAL ENGINEERS, 17 June 2007 (2007-06-17), - 20 June 2007 (2007-06-20) pages 6436-6445, XP008107255 Minneapolis, USA
- DATABASE WPI Week 200420 Thomson Scientific, London, GB; AN 2004-207684 XP002532069 "Manufacturing fatty acid ester and glycerol by reacting alcohol with fats and oil in presence of solid catalyst of calcined lime magnesia lime (mixture of magnesium oxide and calcium oxide) at preset temperature" & JP 2004 035873 A (FIELD TECHNOLOGY KENKYUSHITSU) 5 February 2004 (2004-02-05)
- ASHBY, R.D. ET AL.: "Sophorolipid Biosynthesis from a Biodiesel Co-product Stream" JOURNAL OF THE AMERICAN OIL CHEMISTS' SOCIETY, vol. 82, no. 9, 2005, pages 625-630, XP002532010
- PAPANIKOLAOU, S. ET AL.: "Biotechnological valorisation of raw glycerol discharged after bio-diesel (fatty acid methyl esters) manufacturing process: Production of 1,3-propanediol, citric acid and single cell oil" BIOMASS AND BIOENERGY, vol. 32, no. 1, 2008, pages 60-71, XP022393501
- MEESTERS, P.A.E.P. ET AL.: "High-cell-density cultivation of the lipid accumulating yeast Cryptococcus curvatus using glycerol as a carbon source" APPLIED MICROBIOLOGY AND BIOTECHNOLOGY, vol. 45, no. 5, 1996, pages 575-579, XP002532011

## Description

The present invention concerns a process for forming a lipid or a mixture of lipids from a starting material, which comprises at least one alcohol and a soap or a precursor of a soap. The invention also concerns a process for forming a lipid or a mixture of lipids from an alcohol-containing liquid phase, which comprises at least one alcohol.

### Background of the Invention

Biodiesel is mainly a fatty acid methyl ester, which is formed by transesterification of long-chained fatty acids with an alcohol (methanol). The fatty acid esters of natural fats consist mainly of triglycerides, whereby water-soluble glycerol that is unsuitable as biodiesel is released during the transesterification. Theoretically, 10% by weight of glycerol is generated from the triglyceride. Depending on the process conditions the proportion of the soap can vary greatly and rise up to tens of percent from the initial total amount of triglyceride. Since the soap compounds are dispersed and partially dissolved into the forming aqueous solution of glycerol, separation thereof from the aqueous solution is difficult. Soap disrupts the phase between the fat-soluble fatty acid esters and the water-soluble glycerol and tends to form different degrees of emulsions, creating a challenging problem in large-scale processes with regard to separation techniques. Removal of the alcohol, such as methanol, would also require expensive vacuum distillation. Thus, it can be concluded, that production of biodiesel using the afore-described process in modem technology is a waste of raw material. Increasing the value of the glycerol generated in the process is especially uneconomical already merely with regard to the purification steps required by it.

Some microbes accumulate fat in their cells. It has also been known that glycerol can serve as a carbon source for microbes, even for fat-accumulating microbes *(e.g.* Microbial Lipids, eds. C. Ratledge and S. G. Wilkinson, vol. 1, Academic Press, 1988*;* Papanikolaou, S. and Aggelis, G., Lipid production by Yarrowia lipolytica growing on industrial glycerol in a single-stage continuous culture, Bioresource Technol. 82 (2002) 43-49*.).* On the other hand, during the decades no breakthrough has been created for fat-accumulating microbes and the fat produced by them, except mostly for the small-scale production of some special fats. The average poor quality of this glycerol, e.g. with regard to the alkali metal salts of fatty acids (hereinafter soap) and the alcohol that it contains, has been experienced as a problem in the utilization of the glycerol coming from the biodiesel process, especially in the formation of fat by means of microorganisms. Soap and alcohol prevent the growth of most organisms, and thus their removal is a requisite for microbiological utilization of the glycerol.

A majority of natural fats contain fatty acid containing hydrocarbon chains that are bound to alcoholic groups forming the most energy rich part of the fats. This most energy rich part of fats can be released by forming alcohol esters therefrom. An aqueous solution of glycerol, blended with an alcohol and soap compounds (hereinafter soap) formed from the fatty acids of the fat, is released in the process. This fraction containing glycerol, alcohol and soap is water-soluble and it still has a high energy content especially with regard to the soap. Already with regard to only the glycerol, this water-soluble fraction containing mainly glycerol forms a theoretically over 10% minor waste flow in the production of the fatty acid alcohol esters. Glycerol, as such, is already inexpensive and its markets are already saturated. Thus, there is no inherent energy efficient use within sight for the impure glycerol formed in the production of fatty acid alcohol esters and in view of current technology if forms a cost generating problematic minor flow, for which there is no energy efficient and, thus, profitable technology generating added value.

Commercially interesting possible uses for glycerol or products obtainable therefrom by chemical techniques have been widely investigated. In spite of this, there are no economically durable solutions in sight, by which a substantially higher degree of utilization of glycerol than before would be possible. An extensive summary of the suitability of glycerol as a raw material for the chemical industry and of the challenges associated with the use thereof have been summoned in a research by Sarantila, Maiju: Glyserolin hyödyntäminen, diplomityö, Teknillinen korkeakoulu, 10.11.2006*. [*Sarantila, Maiju: Utilization of Glycerol, Diploma thesis, Helsinki University of Technology, November 10, 2006*].* In the following, based on the thesis, the currently known most essential possible uses of glycerol for increasing the utilization rate and the added value are described.

The oxygen content of the glycerol molecule is relatively high and the carbon content is low, respectively, thus resulting in a poor caloric value. Further, the temperature of a glycerol flame is lower than for traditional fossil fuels. In practice, it is known that combustion of glycerol requires also special arrangements, since it has to be brought to a very small droplet size to obtain sufficient mixing with oxygen. Further, the glycerol released in the production of biofuel contains water.

PYLE, D. & WEN, Z. ("Production of Omega-3 Polyunsaturated Fatty Acid from Biodiesel-Waste Glycerol by Microalgal Fermentation", 2007 ASABE ANNUAL INTERNATIONAL MEETING, TECHNICAL PAPERS, AMERICAN SOCIETY OF AGRICULTURAL AND BIOLOGICAL ENGINEERS, 17 June 2007 (2007-06-17), - 20 June 2007 (2007-06-20), pages 6436-6445) disclose a process of forming a lipid or mixture of lipid in that HCl is used to precipitate and eliminate soap present in the feeding material.

DATABASE WPI, Week 200420, Thomson Scientific, London, GB; AN 2004-207684, "Manufacturing fatty acid ester and glycerol by reacting alcohol with fats and oil in presence of solid catalyst of calcined lime magnesia lime (mixture of magnesium oxide and calcium oxide) at preset temperature" disclose the precipitation of soap with an alkaline earth metal but in the process described no microorganisms are used.

Glycerol can be derivatized chemically in many different ways. For example, the esterification of glycerol, the etherification, the production of acetines and diols are known as well as the polymerization and the oxidation of glycerol. Principally, there are no large scale applications for the products of these processes, especially taking into account that the production thereof requires a high degree of purity of the glycerol and expensive and energy consuming catalyst and reactor technology.

Reforming, i.e. the forming of hydrogen from glycerol, is a technology, by means of which it is possible to treat even large amounts of glycerol. However, reforming requires special apparatuses, copious energy investments and the process itself requires expensive catalysts.

Glycerol has some applications in agriculture. Glycerol can serve as an energy source as a component of animal feed. However, it does not contain protein, fat or carbohydrates. Industrial glycerol is poor even with regard to trace elements, and can thus form only a minor subcomponent in feed mixtures. Further, the glycerol formed in the production of organic fuel contains methanol and soap, which necessitates disproportionate purification operations with regard to the total cost structure of the feed, such as vacuum distillation for the removal of the alcohol or a separate removal of fat.

Thus, it is evident, that economically advantageous solutions do not exist for transforming glycerol and especially heterogenic mixtures containing glycerol as a constituent into commercial products that could significantly increase the demand for glycerol. The significance of this technology deficiency is emphasized especially when the interest towards renewable energy raw material sources alongside fossil raw materials grows.

### Brief description of the invention

The present invention concerns a process for forming a lipid or a mixture of lipids from a starting material, which comprises at least one alcohol and a soap or a precursor of soap.

The present invention also concerns a process for forming a lipid or a mixture of lipids from an alcohol-containing liquid phase, which comprises at least one alcohol, according to which process a lipid-producing microorganism is brought onto a culture substrate, which contains said alcohol-containing liquid phase, the microorganism is allowed to produce lipid, and the cell mass or the lipids produced by the cells are recovered as claimed.

More specifically, the process according to the present invention is characterized by what is stated in the characterizing part of claims 1 and 4.

The invention provides a new solution to a problem relating to a process, wherein esters of fatty acids are produced from lipids, such as glycerolipids, according to an established process, by treating them with alkali metal alkoxides. Glycerol is produced in the process and, in a side reaction, alcohol is released as well as an alkali metal salt, a soap, of water-soluble fatty acids that contain hydrocarbon chains. A central problem of the process is the utilization of the impure glycerol and the purification of the glycerol into a safe and useful compound required for the utilization.

A comprehensive advantage of the invention is that it can be used to produce an energy rich chemical compound, a lipid, by simple process steps, in an energy efficient and environmentally friendly way, from compounds of biological origin containing less energy, such as from a polyhydric alcohol and a monohydric alcohol, preferably avoiding the use of significant amounts of water.

The minor flow containing alcoholic compounds and an unesterified salt of fatty acids, which is difficult to utilize in terms of energy economics and which is released from the transesterification of the fatty acid esters contained in the organic lipids, can be transformed into a fraction that is suitable for the production of glycerolipids consisting of fatty acids, which glycerolipid is utilizable as such or is recyclable into another material of biological origin containing glycerolipid. The treatment of the lipid formed in the microorganism into the desired esters can take place without a previous decomposition of the microorganism cells and a subsequent step of isolation of the fat.

The advantages of the invention include also that the apparatus needed for the process is simple and the technology involved, concerning manufacture and use, is known. The process according to the invention is not bound to production scale, but it can readily be scaled to the amount required by the polyhydric alcohol fraction to be treated. Carrying out the process does not require energy consuming heating, pressurized unit operations or chemical catalysts and it requires, for operating, only the use of such chemicals or the processing of such biomaterials that can be incorporated into the inner circulation of the process according to the invention. The process does also not require cost increasing prepurification of the fraction of polyhydric alcohol, nor removal of water. The total cost-efficiency of the process is improved by the fact that the lipid-free biomass formed during it can, in addition to the inner circulation, be used for many different purposes, such as for the production of single components or as a supplementary culture substrate for producing a microorganism.

Also generally known problems caused by the presence of a surface-active compound can be eliminated by the invention. As is well known, surface-active compounds, such as the soaps of fatty acids, inhibit the growth of microorganisms and serve as a carbon source for only a few microorganisms. The presence of soap in the production process of a microorganism decreases the surface activity of the culture substrate, causes foaming and complicates the separation of the cells from the liquid with methods based on their specific weight. The presence of the soap in the mixture containing water and water-insoluble oil inhibits also the separation of the aqueous and oil phases from each other. The polyhydric alcohol containing soap-like compounds is, thus, poorly suitable for the production of lipid with microbiological means, while the total utilization of the alcoholic minor flow containing the soap generated in the biodiesel process is prevented or weakened. By means of the invention, for example, the organic compounds contained in the glycerol-rich side fraction generated during the production of fatty acid esters can be transformed back into lipids using a process, which combines chemical treatments to achieve microbiological lipid production.

By means of a process according to the invention, a particular flexibility is achieved for microbiologically producing lipid. A fraction containing a polyhydric alcohol without growth-inhibiting harmful components is a natural carbon source for most microorganisms. Thus, an advantage of the process is also that the choice of microorganism can be made within broad ranges, such as based on the capacity of lipid production, the yield of biomass, the means of culturing or the culture conditions. The other components of the microorganism than lipid can be used energy efficiently in many different ways, thus, improving the total cost-efficiency of the process according to the invention.

Thus, compared to the prior art, the invention fulfils the principles of sustainable development by decreasing the total need of fat raw materials from other sources, and has the prerequisites for updating the production costs to a level accepted by consumers.

Next, the invention will be described in more detail with the help of the accompanying drawings and the detailed description.

### Brief description of the drawings

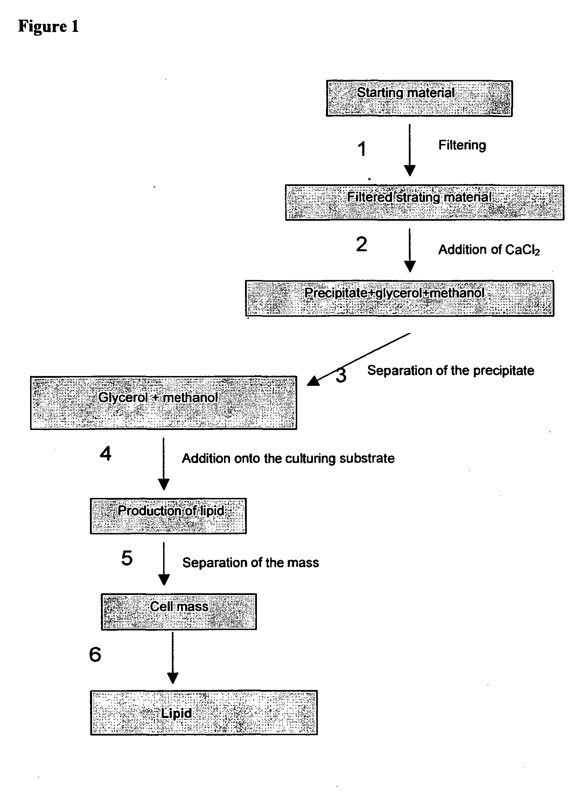

Fig. 1 depicts the main execution steps of the process according the present invention.
Fig. 2 is a graphical presentation of the growth of yeasts on the starting material or the alcohol-containing liquid phase according to the present invention, wherein the present soap has been removed according to the invention.
Fig. 3 is a graphical presentation of the growth of yeasts on pure glycerol (manufacturer J.T.Baker, USA).
Fig. 4 is a graphical presentation of the growth of yeasts on a substrate containing both glycerol and methanol. Fig. 4a illustrates the growth of *Yarrowia lipolytica* yeast on a substrate, which contains 5/10% glycerol either with or without added methanol (2% by weight of the amount of glycerol), and Fig. 4b depicts the growth of *Rhodotorula glutinis* yeast on corresponding substrates.
Fig. 5 is a graphical presentation of the growth of yeasts on a glycerol fraction that contains no soap, which has been used in amounts of 5, 12.5 or 25% on a YNB (Yeast Nitrogen Base) substrate.
Fig. 6 is a graphical presentation of the fat content of the yeasts at different points in time, when the culture substrate (YNB) contains 5 or 12.5% of a biodiesel-glycerol fraction not containing soap. This fat content is shown in Fig. 6a for *R. glutinis* yeast, and in Fig. 6b for *Y. lipolytica* yeast, respectively.
Fig. 7 is a graphical presentation of the growth of *Galactomyces geotrichum* mould on substrates, using glucose (A), pure glycerol (B) or a glycerol fraction not containing soap (C) as a carbon source. Fig. 7a shows the growth curve of the mould and Fig. 7b shows the fat content of the mould cells.

### Detailed description of the invention

The present invention concerns a process for forming a lipid or a mixture of lipids from a starting material, which comprises at least one alcohol and a soap or a soap precursor, characterized by
- adding a mineral salt of an alkaline earth metal to the starting material to transform the soap into a precipitate, whereby a mixture is formed, which contains an insoluble phase and a liquid phase,
- separating the insoluble phase from the liquid phase to recover the precipitate,
- contacting a lipid-producing microorganism with the liquid phase on a culturing substrate,
   whereby the microorganism cells begin producing lipid, and
- collecting the lipids.

Preferably, the starting material comprises a polyhydric or a monohydric alcohol or both, and a soap or a precursor of soap.

The invention also concerns a process for forming a lipid or a mixture of lipids from an alcohol-containing liquid phase, which comprises at least one alcohol, the process further comprising
- contacting the lipid-producing microorganism with the alcohol-containing liquid phase on a culture substrate, whereby the microorganism cells begin producing lipid, and
- collecting the lipids.

Preferably, the liquid phase comprises a polyhydric or a monohydric alcohol or both.

In particular, the present invention concerns a process for forming a lipid or a mixture of lipids from said starting material, where the amount of alcohols is at least half of the starting material, preferably from 70 to 99% by weight, or from the alcohol-containing liquid phase, where the amount of alcohols is from 36 to 100% by weight of the phase, preferably 70 to 100% by weight.

According to a preferred embodiment of the present invention, a fraction comprising the alcohol, formed as a result of the transesterification of the lipid consisting of glycerolipid, carried out using sodium methoxide, is used as a starting material in the process for forming a lipid or a mixture of lipids.

The term "lipid" means a fatty substance, a part of whose molecule is generally an aliphatic hydrocarbon chain that dissolves in organic solvents, but is poorly soluble in water.

In the present invention, the lipids formed in the microorganisms are mainly triacylglycerols, i.e. triesters formed by glycerol and fatty acids (triglycerid), or esters of sterol, but other lipids can also be formed in the cells, such as phospholipids, sterols, polyprenols, sphingolipids, glycolipids and diphosphatidylglycerol.

According to a preferred embodiment of the present invention, triglycerid or a mixture comprising triglycerids, where even large amounts of components, such as sterols and free oxygens, can also be present, is formed from the alcohol-containing liquid phase.

The term "soap" means a salt of a fatty acid.

The invention comprises mainly a process based on natural substages, by which fat (lipid) containing long-chain fatty acid is produced from mixtures of glycerol, other alcohols, such as a monohydric alcohol, and an organic soap. Thus, the invention can be used for transforming highly oxidized organic compounds into reduced lipids having a high energy content, which by further refining are suited for use for different energy production forms.

Next, for simplification, the invention and the embodiments thereof will be described by mentioning glycerol as the polyhydric alcohol, but it is possible to replace it with another polyhydric alcohol suitable for the invention.

Glycerol and monohydric alcohol are suitable as carbon sources for microorganisms, especially since it has been proven, that they, as a mixture, intensify the use of both carbon sources, and by choosing a suitable microorganism these compounds can by means of biosynthesis be transformed into lipids of microorganism-origin consisting of fatty acids and into other biomaterial that can be utilized, such as, as a nutrient inside the process, for isolating commercially interesting compounds or the biomass can be used for other general applications, such as, as feed and nutrient.

The soap that is formed in the acid catalytic manufacturing of fatty acid esters generates problems for the production of a microbe based lipid. Soap slows down or even totally inhibits the growth of the microorganism and thus the biosynthesis of lipids. Soap also complicates the separation of cells from the culture substrate.

According to the process of the present invention, lipid containing fatty acids can be produced microbiologically from impure, soap containing glycerol minor flow by combining chemical and microbiological processes, wherein a fraction containing glycerol and other compounds is used for the production of a lipid-synthesizing microorganism and from which microorganism the thus formed lipid can be used again, for example, for manufacturing alcohol esters of fatty acids.

Chemical and biological reactions suitable for industrial use are combined in a new way in the invention into a process entity, by which the glycerol, the alcohol, which preferably is a monohydric alcohol, and the other organic compounds contained in the glycerol solution, which is formed in the acid catalytic manufacturing of fatty acid esters, can be transformed in an energy efficient way into lipids that contain fatty acids.

The present invention preferably contains steps, wherein the possible solid matter components are removed from the glycerol fraction by filtering, after which the acidity of the glycerol fraction is adjusted with a required amount of acid, preferably with an acid that forms a salt that can serve as a carbon source for a microorganism, more preferably with acetic acid, formic acid or lactic acid. Filtering can be accomplished for example by using a fabric intended for it.

According to a preferred embodiment, the process for forming a lipid or a mixture of lipids comprises the steps of
- optionally adding an acid to the starting material, preferably an organic acid, more preferably acetic acid, formic acid or lactic acid, to adjust the pH to a value of from 3 to 8, preferably to a value of from 6 to 8,
- adding a metal-ion forming agent to the mixture, such as mineral salt of an alkali earth metal, preferably an agent that forms Ca²⁺ or Mg²⁺, more preferably calcium chloride or magnesium chloride, most preferably calcium chloride, as a solid or as a liquid (such as an aqueous solution), in an amount, that precipitates at least 40% of the soap, preferably in an amount, by which a stoichiometric amount of metal ion is formed compared to the amount of soap, most preferably a stoichiometric excess of 10% by weight, whereby an insoluble phase and a liquid phase are formed, wherein the insoluble phase comprises the soap and the liquid phase comprises the alcohol-containing solution containing a polyhydric or a monohydric alcohol, i.e. the aforementioned liquid phase,
- separating the insoluble phase, wherein the possibly present soap precursor has reacted into a soap, is separated from the liquid phase preferably by filtering or by decanting or by another procedure used generally for recovering a precipitate,
- providing the liquid phase and the lipid producing microorganism onto a culture substrate, supplementing the substrate with nutrients required for production of lipid or with disrupted microorganism mass that contains these agents,
- allowing the microorganism cells to produce lipid, whereby the lipid is accumulated inside the microorganism cells or outside them,
- optionally disrupting the microorganism cells,
- optionally separating the lipid by phase separation or extraction,
- recovering the cell mass or the lipids produced by the cells preferably either by filtering or by methods based on differences in specific weight, such as centrifuging, and thereafter by separating the formed fractions, such as by separating the lipid, the lipid-containing fraction or the lipid mixture from the cells of the recovered cell mass, and
- preferably using the cell mass or its fractions as lipids.

Particularly preferably, the water-soluble soap present in the optionally filtered glycerol fraction is transformed into a water-insoluble soap by adding into the mixture a mineral salt of a bivalent alkali earth metal, preferably of Ca²⁺, most preferably CaCl₂. Most suitably, the salt is added as a solid, whereby the use of significant amounts of water can be avoided. The mixing time is controlled, until the formation of a precipitate has essentially stopped. The formed insoluble fraction (the precipitate) is separated from the liquid fraction (the liquid phase) by filtering or by decanting or by other methods generally used for recovering precipitate. The liquid glycerol fraction, with the other compounds it possibly contains, is used by mixing it into the culture substrate of a microorganism in a concentration suitable for its lipid production. After separation of the soap precipitate, the hydrocarbon fractions over C₄ contained by it can be treated in several different ways, preferably for producing free fatty acids, most preferably for producing fatty acid esters.

The compounds to be added in the different stages of the process, such as acetic acid or bivalent alkali earth metal salts, such as CaCl₂ or the side fractions formed by these, do not, apart from NaCl and carbon dioxide, leave the process entirely, but are preferably recycled inside the process or they form separate economically utilizable fractions and, thus, improve the total cost-effectiveness of the process.

By removing surface-active compounds from the alcohol-containing side fraction formed in the transesterification, prerequisites are created for using this fraction for culturing microorganisms and for producing one-cell lipids.

It has, however, surprisingly been found that reagent-pure glycerol is not as such a very efficient carbon source. Methanol, on the other hand, intensifies the use of glycerol, i.e. there is no need for removing methanol, but it can be utilized for producing fat of microbe origin.

The afore-described means that essentially all organic carbon compounds that already are in a relatively oxidized stage from the impure glycerol fraction containing plenty of unutilized hydrocarbon chain can be utilized with such efficiency that microorganisms are capable of utilizing this mixture into lipids.

The polyhydric alcohol used in the invention is chosen from polyhydric aliphatic alkyl alcohols containing at least 3 carbons. Preferably, the polyhydric alcohol is glycerol, an alcohol formed form phospholipids or a sterol. Most suitably the polyhydric alcohol is glycerol. The monohydric alcohol, on the other hand, is chosen from alkyl alcohols containing 1 to 4 carbons, preferably methanol, ethanol or 1-propanol. Most suitably, the monohydric alcohol is methanol.

The microorganism is chosen from natural or modified, fat accumulating microorganisms, preferably from yeasts, moulds, bacteria and algae, more preferably from yeasts and moulds, most suitably from yeast. It is essential that said microorganism is capable of producing lipid.

The fat accumulating yeast genera suitable for the invention comprise the following:
- *Candida* (inter alia C. *curvata*)
- *Yarrowia* (inter alia *Y. lipolytica*)
- *Lipomyces* (inter alia *L. starkeyi*)
- *Rhodotorula* (inter alia *R. glutinis)*

Correspondingly, the fat accumulating mould genera suitable for the invention comprise the following:
- *Mortierella*
- *Muco*
- *Galactomyces*

Correspondingly, the fat accumulating bacterium genera comprise the following:
- *Rhodococcus*
- *Uscillatoria*
Likewise, the fat accumulating microalgae genera comprise the following:
- *Crypthecodiniumi*
- *Ulkenia*
- *Schizochytrium*

According to a preferred embodiment of the invention, microorganisms that synthesize fatty acid -containing lipid into their cells in an amount, which preferably is from 12 to 60% by weight of the dry weight of the cells are used for the synthesis of lipids.

According to another preferred embodiment of the invention, microorganisms capable of using glycerol and short-chained alcohols as a carbon source are used for the synthesis of lipids.

According to a particularly preferred embodiment of the invention, the lipid-free biomass formed in the invention, treated in a way suitable for the microorganism, is used as nutrients for the culture substrate. The culture substrate can in addition to these components be supplemented with components that are advantageous for the used microorganism. In order to produce lipid, the microorganism generally needs, inter alia, a carbon source, which it in the present invention obtains from the starting material, a nitrogen source, such as an inorganic ammonium salt (e.g. ammonium sulphate) or an organic nitrogen source (e.g. amino nitrogen, yeast extract, or hydrolyzed cell mass), and a source of trace elements, such as a source of phosphates, sulphates, chlorides, vitamins or cations (e.g. a source of Mg, K, Na, Ca, Fe or Cu ions), whereby these components can be added onto the substrate, if necessary. When applying the process of the invention, the alcohol content of the culture substrate is preferably from 2 to 36% by weight.

In order to recover the lipids from the microorganism, the cells are first collected and then they are either disrupted or they disrupt for example as a result of autolysis, whereby the lipids are separated from the aqueous phase as an oil phase, or, alternatively, the microorganism mass as such or a fraction obtained from it by different known methods is used as the lipid. The fatty acid containing lipid, which is contained in the microorganisms or produced by them, can be transesterified without a previous separation of the lipids from the cells or the cells are disrupted and the lipid is extracted from the crushed cells with an organic solvent. The methods of recovery of lipid that are suitable for the invention are described for example in the publication by Z. Jacob: Yeast Lipids: Extraction, Quality Analysis, and Acceptability, Critical Reviews in Biotechnology, 12(5/6); 463-491 (1992).

The amount of lipid forming in the cells can increase, when using the present process, into up to 60% of the dry matter of the cells.

The lipid or the mixture of lipids that is produced according to the process of the present invention can be utilized in many different applications. Preferably, esterification, such as transesterification, or hydrotreating, is carried out for the lipids, the lipid mixture, the lipid-containing cells or a fraction thereof.

According to a particularly preferred embodiment of the present invention, the produced lipid is used for the production of alcohol esters of fatty acids. More suitably, these alcohol esters of fatty acids are further used in the manufacturing of biofuels, such as biodiesel. Even more preferably, these fatty acid esters are used further in the manufacturing of biodiesel (methyl or ethyl ester) or renewable diesel (hydrotreated lipid of animals, plants or microbes, whereby the microbe lipid can be derived from bacteria, yeast, mould, alga or another microorganism). Correspondingly, the starting material used in the process of the present invention is preferably obtained from the methanol-containing glycerol fraction formed in the manufacturing of, e.g., biodiesel, which generally contains about 2 to 10% of soap. The amount of water (and monohydric alcohol, such as methanol) is minimized in the reaction, since water causes saponification. Less than 20%, preferably 2 to 10% of water is released into said methanol-containing glycerol fraction. This water content is preferred in the starting material used in the process of the present invention.

Vegetable oil and animal fats comprising triglycerides of fatty acids have generally been used as raw material for diesel fuels. Transformation of these raw materials into fuel comprises processes such as transesterification, catalytic hydrotreating, hydro cracking, catalytic cracking and heat cracking. Typically, triglycerides have not been used as such, but they have been transformed into the respective fatty acid esters in a transesterification reaction.

The product of the present invention is suitable as, for example, a raw material in the last-mentioned reaction, preferably as a feed in the esterification of fatty acids or in processes, wherein lipids of plant or animal origin are hydrotreated, most preferably as a feed in processes, wherein so called HVO ("hydrogenated vegetable oil") is manufactured.

The products according to the present invention, to be used in transesterifications, preferably contain double bonds in order to achieve advantageous cold flow characteristics for the end product, i.e. the fuel.

The lipids forming in the present invention can contain 0 to 40% by weight of free fatty acids, which are formed, for example in the enzymatic hydrolysis of triglycerides or in connection with the purification of fats. Also these fatty acids can be utilized in the manufacturing of biofuels, either as such, or the acids and the triglycerides can be esterified into methyl esters, which, on the other hand, can be used in biodiesel.

According to EU-directive 2003/30/EY, "biodiesel" means "a methyl-ester produced from vegetable or animal oil, of diesel quality to be used as biofuel".

The biomass formed from the microorganism, remaining after the separation of lipids, is treatable and utilizable in many different ways, such as by using it over again in the culture substrate needed for the growth of the microorganism, as such or after an enzymatic, chemical of physical, including mechanical, processing. Alternatively, the lipid-free cell mass is divided into different constituent fractions. A preferred alternative is to carry out an acid catalytic hydrolysis directly on the cell mass, after which the lipid can be separated from the other hydrolyzed cell mass, for example by phase separation, and the remaining biomass can be used for culturing the microorganism. Another alternative is using the lipid-free cell mass for isolation of commercially significant components. These include proteins, protein hydrolysates, peptides derived from proteins, and, especially when using yeast, in addition the aforementioned, beta-glucans, xanthans, vitamins, vitamin precursors and sterols. A preferred application of the lipid is the use of the lipid-containing microorganism mass as such as a lipid raw material for the production of fatty acid esters.

The following examples are meant to illustrate the invention, and they should not be considered in any way as limiting to the invention. The invention is also by no means restricted to the strains of microorganisms used. The invention can be carried out, in addition to the used strains, also with the help of other strains of the same species or genus. Lipid producing microorganisms (also algae) are generally available and they can be found in several strain collections, for example ATCC, DSM etc. Lipid producing microorganisms and lipid production processes with microorganisms (also with algae), have been described in the literature, for example in the works Single Cell Oils, eds. Z. Cohen and C. Ratledge, AOCS Press, 2005 and Microbial Lipids, eds. C. Ratledge and S.G. Wilkinson, vol. 1 and 2, Academic Press, 1988.

### Examples

### Example 1. Growth of yeast on a starting material according to the invention

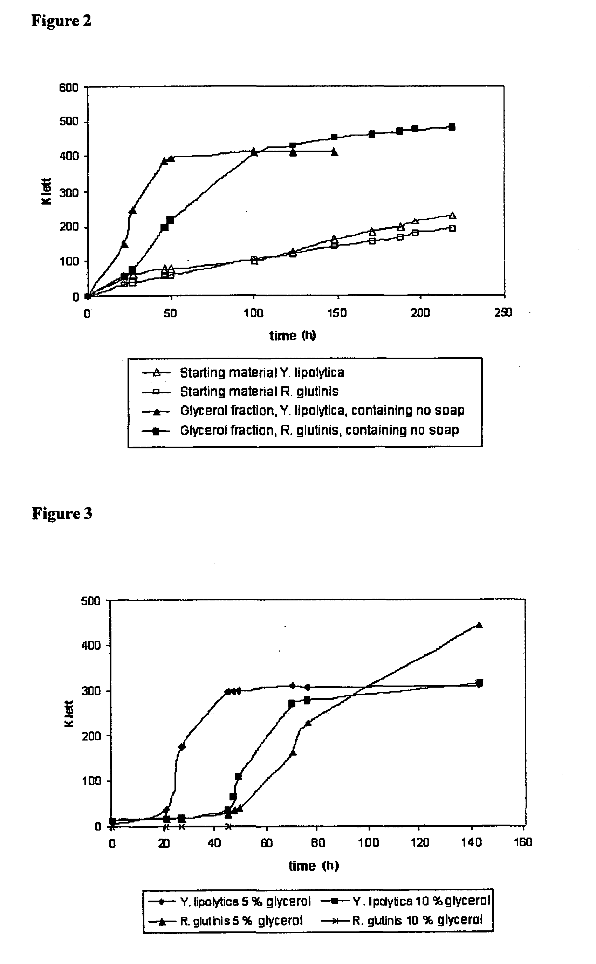

*Yarrowia lipolytica* ATCC 20373 and *Rhodotorula glutinis* TKK 3031 yeasts were cultured on a YNB substrate supplemented with 5% (weight/volume) of a soap-containing biodiesel glycerol fraction, i.e. the starting material of the invention, or a biodiesel glycerol fraction, from which the soap has been removed according to the invention, they were shaken 250 rpm at 30°C and the growth was monitored by means of increase of opacity with a Klett-Summerson colorimeter. The growth curves are shown in Fig. 2. It can be seen from the Figure that the yeasts grew very poorly on the soap-containing glycerol fraction. It is noteworthy, that the growth of the yeast cultured on the glycerol fraction not containing soap seems almost linear in respect of the culturing time, which is atypical for this culturing manner, and the increase of opacity may be a result of the emulsion produced by the soap with the other components of the mixture. The recovery of the cells was not possible even by centrifuging, which reveals that the remaining amount of cells was associated with the soap and these together resisted the gravitational force of the centrifugation through the phase separation. Consequently, the fat content of the cells could also not be determined.

The experiment depicted above shows that the glycerol fraction formed in the acid catalytic manufacture of fatty acid ester (the soap-containing biodiesel-glycerol fraction) is not suitable for microbiological production of glycerolipids.

### Example 2. Growth of yeast on pure glycerol

The yeasts of Example 1 were cultured on a YNB substrate having as a carbon source 5 or 10% (w/v) of pure (by over 99%) glycerol (manufacturer J.T.Baker, USA). The culturing was performed at room temperature and with 250 rpm shaking. The growth curve of the yeasts is shown in Fig. 3. The yeasts grew either very slowly or only to a moderate cell content. Particularly *R. glutinis* yeast grew poorly and on 10% glycerol it did not grow at all.

Thus, it can be concluded, that with respect to that fraction of glycerol, from which the soap has been removed according to the invention, pure glycerol containing no methanol is not an efficient carbon source for the production of a microorganism mass and, thus, for the production of glycerolipids.

### Example 3. The effect of methanol on the growth

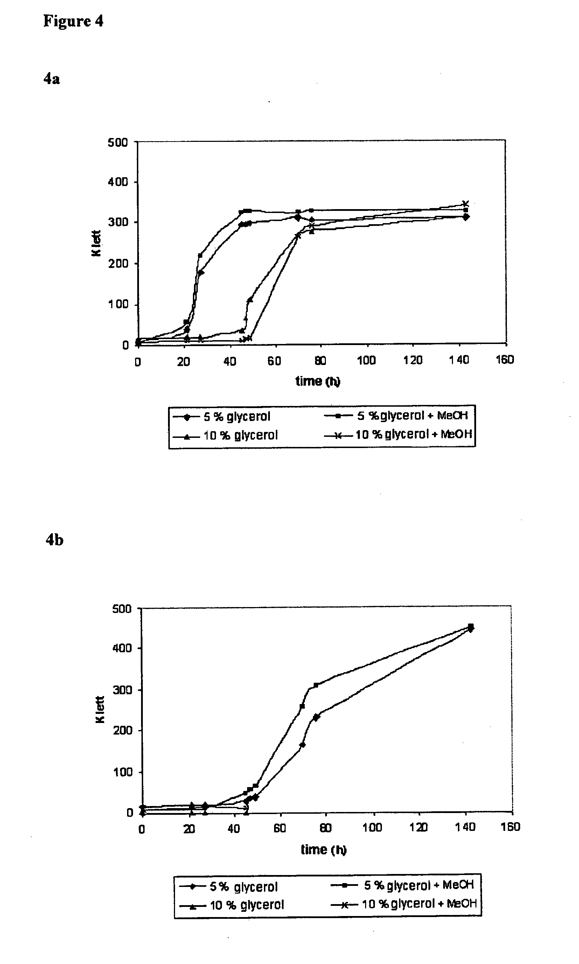

When 2% by weight methanol of the amount of glycerol was added to the substrate according to Example 2 and the growth of the yeasts was monitored, it was observed that the presence of methanol either boosted the growth of the yeast or did not affect the growth at all (Fig. 4).

Thus, these experiments show that the presence of an alcohol in the glycerol fraction is advantageous with respect to the complete use of the glycerol fraction.

### Example 4. The growth of yeast and the production of fat on a glycerol fraction, from which the soap has been removed according to the invention

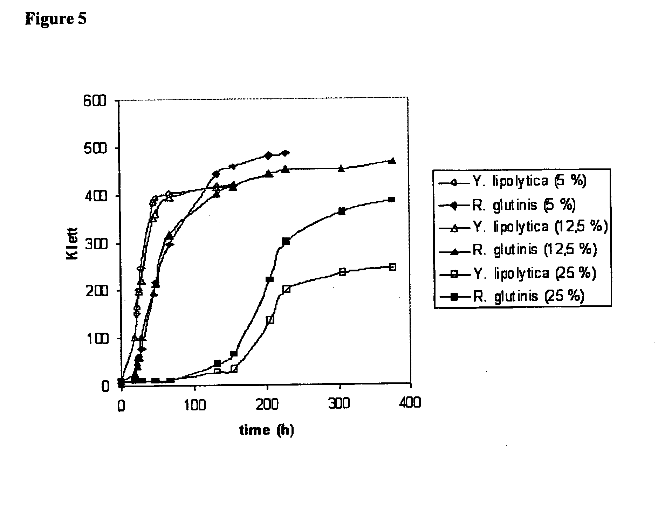

*Y. lipolytica* and *R. glutinis* yeasts were grown on a YNB substrate supplemented with 5; 12.5 or 25% (w/v) of a glycerol fraction (from which the soap had been removed). The culturing took place at room temperature with shaking at 250 rpm. The growth curves are shown in Fig. 5. It is seen from the figure that the yeasts grew rapidly and almost as well on substrates containing 5 and 12.5% of glycerol fraction, but the 25% glycerol fraction slowed down the growth clearly. In this experiment also *R. glutinis* grew on the substrate that contained 12.5% of the glycerol fraction. However, it should be noted that in the case of the substrates that contained 5% of glycerol fraction, 2% of displacement was used, whereas the size of the displacement with higher glycerol concentrations was 1%. This is, however, not believed to have an effect on the achieved cell concentration or on the amount of fat.

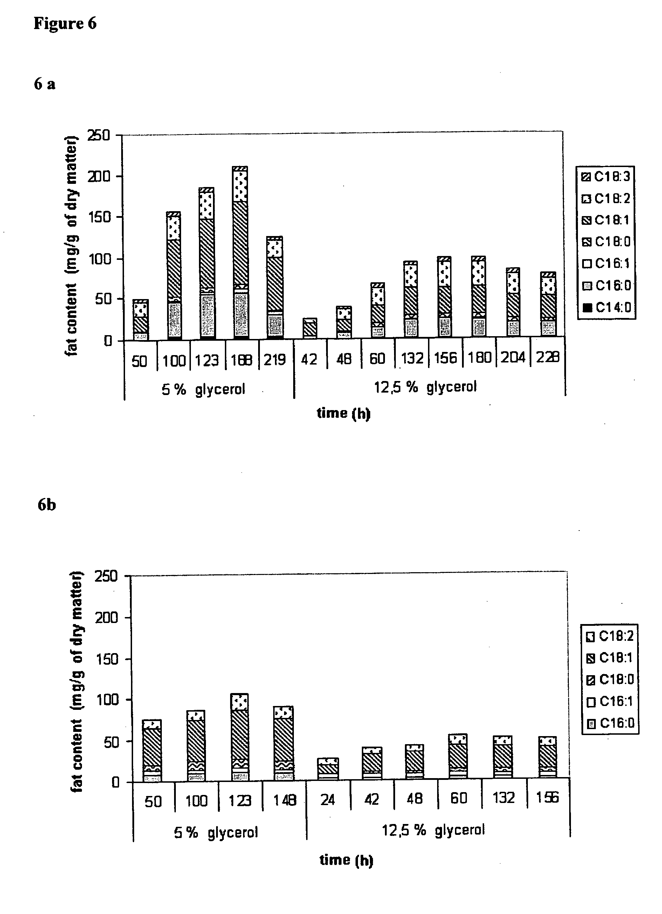

The fat content was determined from the cells cultured on biodiesel-glycerol containing no soap (5/12.5% of glycerol fraction) at different points in time by gas chromatography. The results are shown in Figs. 6a-b. It can be seen from the figures that the fat content of the yeast cells grew higher with a lower glycerol concentration. The *R. glutinis* yeast contained considerably more fat than the *Y. lipolytica* yeast at the investigated conditions.

### Example 5. Culturing of mould on glycerol containing no soap

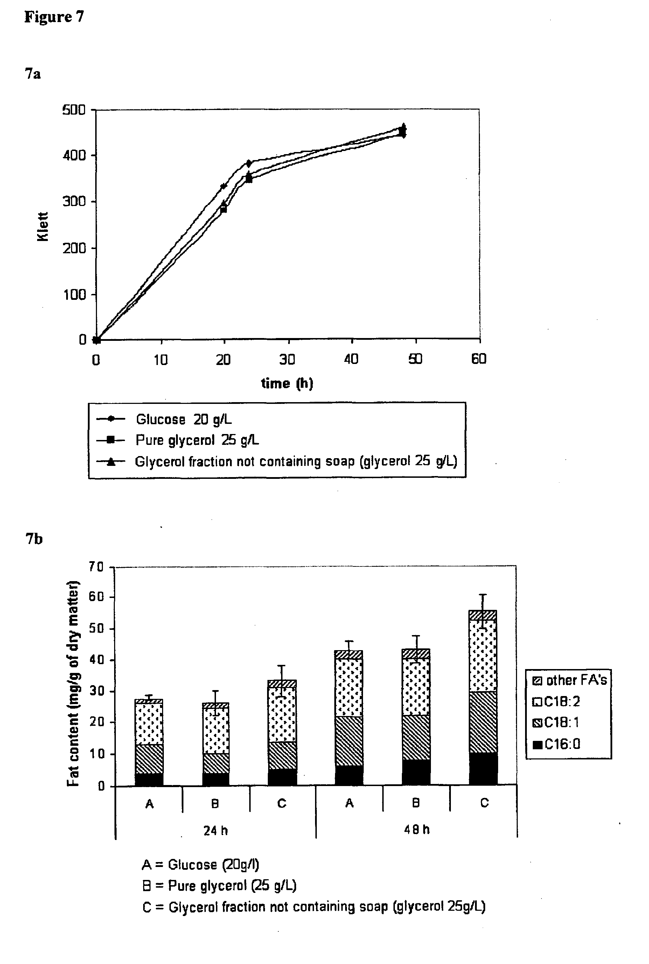

*Galactomyces geotrichum (Geotrichum candidum)* DSM 1240 mould was cultured on 50 ml of a culturing substrate (yeast extract 3 g/l, KH₂PO₄ 3 g/l, MgSO₄ 5 g/l) containing the biodiesel glycerol fraction, from which the soap had been removed according to the invention, or pure glycerol (J.T. Baker) 25 g/l. In the comparative experiment, glucose (20 g/l) served as a carbon source. The cells were cultured at a temperature of 20°C with 200 rpm shaking for 48 hours. The growth was monitored with a Klett-Summerson colorimeter. The growth curves of the cells cultured on different carbon sources are shown in Fig. 7a. It can be seen from the figure that the biodiesel-glycerol, from which the soap had been removed according to the invention, served as an excellent carbon source for the mould, and no significant difference was observed in the growth rate of the cells compared to the growth rate of the cells cultured on glucose. The methanol contained in the biodiesel glycerol fraction was not observed to have any inhibiting effect on the mould growth, although neither any promoting effect.

The fat content of the mould cells was determined at time points of 24 and 48 hours. It becomes evident from Fig. 7b that the biodiesel glycerol fraction as a carbon source resulted in a higher fat content in the cells than with other investigated carbon sources (at 48 hours the difference was statistically significant P<0.005). Further, it must be noted that by extending the culturing time the fat content of the mould cells would probably have risen even higher.

### Example 6. Culturing algae on glycerol

A diatom *Phaeodactylum tricornutum* was cultured mixotrophically using a biodiesel-glycerol fraction, from which the soap had been removed according to the invention, as the carbon source fraction. Pure glycerol (manufacturer J.T. Baker) served as a control carbon source. The concentration of glycerol in the culturing substrate (sterilized sea water) was 8 g/l. The cultures were carried out in 1 liter Erlenmeyer flasks so that the cultures were stirred by means of air blown through a sterile filter and illuminated continuously with a fluorescent lamp. The cells were cultured for 250 hours, after which the cell yield for the biodiesel-glycerol fraction was 2.0 g of dry cell mass /l and for the purified glycerol 1.8 g/l. The obtained fat content of the cells was, for the biodiesel-glycerol fraction, 121 mg/g of dry matter, and for the purified glycerol, 128 mg/g of dry matter.

## Claims

1. A process for forming a lipid or a mixture of lipids from a starting material, which comprises at least one alcohol and a soap or a soap precursor, **characterized by**
- adding a mineral salt of an alkaline earth metal to the starting material to transform the soap into a precipitate, whereby a mixture is formed, which contains an insoluble phase and a liquid phase,
- separating the insoluble phase from the liquid phase to recover the precipitate,
- contacting a lipid-producing microorganism with the liquid phase on a culturing substrate, whereby the microorganism cells begin producing lipid, and
- collecting the lipids.

2. The process according to claim 1, **characterized in that** the starting material comprises a polyhydric or a monohydric alcohol or both, and a soap or a precursor of a soap.

3. The process according to claim 1 or 2, **characterized in that** the amount of alcohol is at least half of the starting material.

4. The process according to any of claims 1 to 3, **characterized in that** the amount of alcohol is 36-100 % by weight of the liquid phase.

5. The process according to any preceding claim, **characterized in that** the polyhydric alcohol is glycerol, an alcohol formed from phospholipids or a sterol.

6. The process according to any preceding claim, **characterized in that** the monohydric alcohol is methanol, ethanol or 1-propanol.

7. The process according to any preceding claim, **characterized in that** the insoluble phase comprises the soap.

8. The process according to any preceding claim, **characterized in that** an acid is added to the mixture before addition of the metal-ion forming agent to adjust the pH to a value of 3 to 8.

9. The process according to claim 6, **characterized in that** the acid is an organic acid selected from acetic acid, formic acid or lactic acid.

10. The process according to any preceding claim, **characterized in that** the mineral salt of an alkaline earth metal is calcium chloride or magnesium chloride.

11. The process according to any preceding claim, **characterized in that** the mineral salt of an alkaline earth metal is added as a solid or as a liquid.

12. The process according to any preceding claim, **characterized in that** the mineral salt of an alkaline earth metal is added in an amount that precipitates at least 40% of the soap

13. The process according to any preceding claim, **characterized in that** the microorganism is a yeast, a mould, a bacterium or an alga.

14. The process according to any preceding claim, **characterized by** collecting the cell mass of the microorganisms or the lipids produced by the cells, after which the lipid, the lipid-containing fraction or the mixture of lipids are preferably separated from the cells of the collected cell mass.

15. The process according to any preceding claim, **characterized by** carrying out an esterification or a hydrotreating on the lipid, the lipid mixture, the lipid-containing cells or the fraction thereof.

16. The process according to any preceding claim, **characterized by** reutilizing the collected cell mass or the cell mass, from which the lipids have been separated, in a culturing substrate.

17. The process according to any preceding claim, **characterized by** carrying out an enzymatic, a chemical or a physical processing on the cell mass before utilizing it.

18. The process according to any preceding claim, **characterized by** separating components, such as proteins, protein hydrolysates, peptides originating from proteins, beta-glucan, xanthans, vitamins, vitamin precursors or sterols or several of these, from the lipid-containing cells.

## Patentansprüche

1. Ein Verfahren zum Ausbilden eines Lipids oder eines Gemischs von Lipiden aus einem Ausgangsmaterial, welches zumindest einen Alkohol und eine Seife oder einen Seifenpräkursor aufweist, gegenzeichnet durch
- Zufügen eines Mineralsalzes eines Erdalkalimetalls zu dem Ausgangsmaterial, um die Seife in eine Ausfällung umzuwandeln, wodurch ein Gemisch ausgebildet wird, welches eine unlösliche Phase und eine flüssige Phase enthält,
- Trennen der unlöslichen Phase von der flüssigen Phase, um die Ausfällung zurückzugewinnen,
- Kontaktieren eines Lipid produzierenden Mikroorganismus mit der flüssigen Phase an einem Kultivierungssubstrat, wodurch die Mikroorganismus-Zellen beginnen, Lipid zu produzieren, und
- Sammeln der Lipide.

2. Das Verfahren gemäß Anspruch 1, **dadurch gekennzeichnet, dass** das Ausgangsmaterial einen mehrwertigen oder einen einwertigen Alkohol oder beides und eine Seife oder einen Präkursor einer Seife aufweist.

3. Das Verfahren gemäß Anspruch 1 oder 2, **dadurch gekennzeichnet, dass** die Menge von Alkohol zumindest die Hälfte des Ausgangsmaterials ist.

4. Das Verfahren gemäß einem der Ansprüche 1 bis 3, **dadurch gekennzeichnet, dass** die Menge von Alkohol 36-100 Gewichts-% der flüssigen Phase ist.

5. Das Verfahren gemäß einem vorangehenden Anspruch, **dadurch gekennzeichnet, dass** der mehrwertige Alkohol Glycerol, ein Alkohol, welcher von Phospholipiden ausgebildet ist, oder ein Sterol ist.

6. Das Verfahren gemäß einem vorangehenden Anspruch, **dadurch gekennzeichnet, dass** der einwertige Alkohol Methanol, Ethanol oder 1-Propanol ist.

7. Das Verfahren gemäß einem vorangehenden Anspruch, **dadurch gekennzeichnet, dass** die unlösliche Phase die Seife aufweist.

8. Das Verfahren gemäß einem vorangehenden Anspruch, **dadurch gekennzeichnet, dass** eine Säure zu dem Gemisch vor Zufügen des Metallionen ausbildenden Mittels zugefügt wird, um den pH auf einen Wert von 3 bis 8 einzustellen.

9. Das Verfahren gemäß Anspruch 6, **dadurch gekennzeichnet, dass** die Säure eine organische Säure ist, welche aus Essigsäure, Ameisensäure oder Milchsäure ausgewählt ist.

10. Das Verfahren gemäß einem vorangehenden Anspruch, **dadurch gekennzeichnet, dass** das Mineralsalz eines Erdalkalimetalls Calciumchlorid oder Magnesiumchlorid ist.

11. Das Verfahren gemäß einem vorangehenden Anspruch, **dadurch gekennzeichnet, dass** das Mineralsalz eines Erdalkalimetalls als ein Feststoff oder als eine Flüssigkeit zugefügt wird.

12. Das Verfahren gemäß einem vorangehenden Anspruch, **dadurch gekennzeichnet, dass** das Mineralsalz eines Erdalkalimetalls in einer Menge zugefügt wird, welche zumindest 40% der Seife ausfällt.

13. Das Verfahren gemäß einem vorangehenden Anspruch, **dadurch gekennzeichnet, dass** der Mikroorganismus eine Hefe, ein Schimmelpilz, ein Bakterium oder eine Alge ist.

14. Das Verfahren gemäß einem vorangehenden Anspruch, **gekennzeichnet durch** Sammeln der Zellmasse der Mikroorganismen oder der Lipide, welche von den Zellen produziert wurden, wonach das Lipid, die Lipid enthaltende Fraktion oder das Gemisch von Lipiden von den Zellen der gesammelten Zellmasse bevorzugt getrennt wird.

15. Das Verfahren gemäß einem vorangehenden Anspruch, **gekennzeichnet durch** Ausführen einer Veresterung oder eines Hydrotreatings des Lipids, des Lipid Gemischs, der Lipid enthaltenden Zellen oder der Fraktion davon.

16. Das Verfahren gemäß einem vorangehenden Anspruch, **gekennzeichnet durch** Wiederverwendung der gesammelten Zellmasse oder der Zellmasse, von welcher die Lipide getrennt wurden, in einem Kultivierungssubstrat.

17. Das Verfahren gemäß einem vorangehenden Anspruch, **gekennzeichnet durch** Ausführen einer enzymatischen, einer chemischen oder einer physikalischen Verarbeitung an der Zellmasse vor deren Verwendung.

18. Das Verfahren gemäß einem vorangehenden Anspruch, **gekennzeichnet durch** Trennen von Bestandteilen, wie z.B. Proteine, Protein-Hyrolysate, Peptide, welche von Proteinen stammen, Beta-Glucan, Xanthane, Vitamine, Vitamin Präkursoren oder Sterole oder verschiedene von diesen, von den Lipid enthaltenden Zellen.

## Revendications

1. Procédé de formation d'un lipide ou d'un mélange de lipides à partir d'un matériau de départ, qui comprend au moins un alcool et un savon ou un précurseur de savon, **caractérisé par**
- l'ajout d'un sel minéral d'un métal alcalino-terreux au matériau de départ pour transformer le savon en un précipité, moyennant quoi un mélange est formé, lequel contient une phase insoluble et une phase liquide,
- la séparation de la phase insoluble de la phase liquide pour récupérer le précipité,
- la mise en contact d'un microorganisme producteur de lipides avec la phase liquide sur un substrat de culture, moyennant quoi les cellules du microorganisme commencent la production de lipides, et
- la collecte des lipides.

2. Procédé selon la revendication 1, **caractérisé en ce que** le matériau de départ comprend un alcool polyhydrique ou monohydrique ou les deux, et un savon ou un précurseur d'un savon.

3. Procédé selon la revendication 1 ou 2, **caractérisé en ce que** la quantité d'alcool est d'au moins la moitié du matériau de départ.

4. Procédé selon l'une quelconque des revendications 1 à 3, **caractérisé en ce que** la quantité d'alcool est de 36 à 100 % en poids de la phase liquide.

5. Procédé selon l'une quelconque des revendications précédentes, **caractérisé en ce que** l'alcool polyhydrique est le glycérol, un alcool formé à partir des phospholipides ou un stérol.

6. Procédé selon l'une quelconque des revendications précédentes, **caractérisé en ce que** l'alcool monohydrique est le méthanol, l'éthanol ou le 1-propanol.

7. Procédé selon l'une quelconque des revendications précédentes, **caractérisé en ce que** la phase insoluble comprend le savon.

8. Procédé selon l'une quelconque des revendications précédentes, **caractérisé en ce qu'**un acide est ajouté au mélange avant l'ajout de l'agent formant un ion métallique pour ajuster le pH à une valeur de 3 à 8.

9. Procédé selon la revendication 6, **caractérisé en ce que** l'acide est un acide organique choisi parmi l'acide acétique, l'acide formique ou l'acide lactique.

10. Procédé selon l'une quelconque des revendications précédentes, **caractérisé en ce que** le sel minéral d'un métal alcalino-terreux est le chlorure de calcium ou le chlorure de magnésium.

11. Procédé selon l'une quelconque des revendications précédentes, **caractérisé en ce que** le sel minéral d'un métal alcalino-terreux est ajouté sous forme de solide ou de liquide.

12. Procédé selon l'une quelconque des revendications précédentes, **caractérisé en ce que** le sel minéral d'un métal alcalino-terreux est ajouté dans une quantité qui précipite au moins 40 % du savon.

13. Procédé selon l'une quelconque des revendications précédentes, **caractérisé en ce que** le microorganisme est une levure, une moisissure, une bactérie ou une algue.

14. Procédé selon l'une quelconque des revendications précédentes, **caractérisé par** la collecte de la masse de cellules des microorganismes ou des lipides produits par les cellules, après quoi on sépare de préférence le lipide, la fraction contenant le lipide ou le mélange de lipides des cellules de la masse de cellules récoltée.

15. Procédé selon l'une quelconque des revendications précédentes, **caractérisé par** la réalisation d'une estérification ou d'un hydrotraitement du lipide, du mélange de lipides, des cellules contenant le lipide ou de leur fraction.

16. Procédé selon l'une quelconque des revendications précédentes, **caractérisé par** la réutilisation de la masse de cellules récoltée ou de la masse de cellules, de laquelle les lipides ont été séparés, dans un substrat de culture.

17. Procédé selon l'une quelconque des revendications précédentes, **caractérisé par** la réalisation d'un traitement enzymatique, chimique ou physique sur la masse de cellules avant son utilisation.

18. Procédé selon l'une quelconque des revendications précédentes, **caractérisé par** la séparation des composants, tels que des protéines, des hydrolysats de protéine, des peptides issus de protéines, le bêta-glucane, des xanthanes, des vitamines, des précurseurs de vitamines ou des stérols, ou plusieurs d'entre eux, à partir des cellules contenant le lipide.
